(19) Europäisches Patentamt
European Patent Office
Office européen des brevets

(11) Publication number : 0 329 195 B1

# (12) EUROPEAN PATENT SPECIFICATION

(45) Date of publication of patent specification : 08.05.91 Bulletin 91/19

(21) Application number : 89105399.3

(22) Date of filing : 22.12.83

(51) Int. Cl.[5] : C10M 133/38, C10M 133/40, C10M 133/58, C10L 1/22, // C10N10:00, C10N30:04, C10N60:00, C10N60:06, C10N60:10, C10N60:12, C10N60:14

## (54) Polycyclic polyamine multifunctional lubricating oil additives.

(30) Priority : 27.12.82 US 453143
16.11.83 US 550977

(43) Date of publication of application : 23.08.89 Bulletin 89/34

(61) Publication number of the earlier application in accordance with Art. 76 EPC : 0113582

(45) Publication of the grant of the patent : 08.05.91 Bulletin 91/19

(84) Designated Contracting States : BE DE FR GB IT NL

(56) References cited :
US-A- 3 112 315
US-A- 3 219 666

(73) Proprietor : EXXON RESEARCH AND ENGINEERING COMPANY
P.O.Box 390, 180 Park Avenue
Florham Park, New Jersey 07932 (US)

(72) Inventor : Brois, Stanley James
773 Lamberts Mill Road
Westfield New Jersey (US)
Inventor : Gutirrez, Antonio
22 Tar Heels Road
Mercerville New Jersey (US)

(74) Representative : Northover, Robert Frank et al
Exxon Chemical Limited Exxon Chemical Technology Centre PO Box 1
Abingdon Oxfordshire, OX13 6BB (GB)

EP 0 329 195 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel lubricating oil additives and lubricating oil compositions containing same which exhibit the desirable properties of being multifunctional dispersants and viscosity index improvers for both gasoline and diesel engine formulations. More particularly, the invention relates to amine derivatives of oil soluble hydrocarbon substituted succinic anhydrides which are characterized by a polycyclic polyamine structure resulting from reaction with certain pre-formed cyclic amino compounds.

Lubricating oil additives, especially dispersants prepared by the reaction of hydrocarbon substituted succinic acid anhydrides with polyamines, to give linear mono and bis-imides, are well known in the art and representative disclosures include US-A-3 172 892 and US-A-3 272 746. Patents which disclose a cyclic structure for the dispersant additive include US-A-4 102 798 and US-A-4 239 636 which discloses a product containing a macrocyclic lactone ester.

Dispersant viscosity index (VI) improvers which result from the grafting of maleic anhydride to an ethylene propylene copolymer with subsequent reaction with polyamines are also known and are disclosed ; for example, in US-A-4 089 794 and US-A-4 144 181. The present invention is considered distinguished from the foregoing patents through formation of the characteristic macrocylcic polyamine structure.

US-A-4 160 739 teaches that in the reaction between polyamines and grafted maleic anhydride polymers, only one reactive amine group must be present in the polyamine to eliminate interfering reactions. US-A-4 137 185 teaches that, in the reaction between the same materials, alkylene polyamines having two primary amine groups produce linear amino-imides which can cause adverse increases in viscosity, and, therefore, acylation of $NH_2$ groups in the amino-imide products with anhydrides is required to prevent chain extension.

One aspect of the present invention is based upon the discovery that certain polycylic polyamine compounds possess significant properties as multi-functional dispersant-viscosity index improvers, especially the capability to be a highly effective dispersant in both a gasoline engine lubricating oil formulation and corresponding diesel engine lubricating oil formulations and thereby enable lubricating oils containing these novel additives to pass or exceed the highest qualification standards for such oils.

According to the present invention polycyclic polyamine additives can be prepared by condensing hydrocarbon succinic anhydrides with polycyclic polyamines (aza polycycles) in an acylation reaction.

The hydrocarbon substituted succinic anhydrides which are suitable for reaction with the pre-formed cyclic amines in accordance with this invention are derived generally from oil soluble hydrocarbons comprising unbranched saturated or unsaturated hydrocarbon chains of at least 8, preferably at least 50 carbon atoms including both polymeric, oligomeric and nonpolymeric aliphatic chains, particularly polymers of $C_2$-$C_5$ olefins. Particularly preferable for use in this invention is thermal polyisobutenyl succinic anhydride of Mn about 900 to 2,000 produced in the "ene" reaction by heating together polyisobutylene and maleic anhydride at about 200°C. Hydrocarbon substituents having from about 8 to 50 carbon atoms, while forming useful oil-soluble products, are less preferable as dispersant viscosity index improvers. They have been found, however, to exhibit desirable properties as antioxidants in oleaginous compositions, such as products of the present invention derived from octadecenyl succinic anhydride.

Suitable olefin polymers include polymers comprising a major molar amount of $C_2$ to $C_5$ mono olefins, e.g., ethylene, propylene, butylene, isobutylene and pentene. The polymers may be homopolymers such as polyisobutylene, as well as copolymers of two or more such olefins such as copolymers]of ethylene and propylene, butylene and isobutylene, propylene and isobutylene and the like. Other copolymers include those in which a minor molar amount of the copolymer monomers, e.g, 1-20 mole%, is a $C_4$ to $C_{18}$ non-conjugated diolefin, e.g., a copolymer of ethylene, propylene and 1,4-hexadiene, i.e. the EPDM polymers.

The olefin polymers will usually have number average molecular weight within the range of about 500 to about 200,000 more usually to about 60,000. Particularly useful olefin polymers for dispersant additives have number average molecular weights (Mn) within the range of about 900 to about 2,000 with approximately one terminal bond per polymer chain. Especially useful material in the present invention is polyisobutylene. Polybutene-1 and polypropylene are also preferred hydrocarbon substituents for preparing dispersants.

Particularly useful materials, when dispersant-viscosity index improving additives are desired, are derived from ethylene/propylene copolymers, terpolymers and tetrapolymers having a $M_n$ of about 10,000 to 200,000, especially 25,000 to 100,000. An example is a copolymer of about 30 to 85 mole% ethylene, 15 to 70 mole% $C_3$ to $C_5$ mono-alphaolefin, preferably propylene, and 0 to 20 mole% of a $C_4$ to $C_{14}$ non-conjugated diene. Preferred examples of such dienes include those of U.S. Pats. 3,790,480 ; 4,089,794 and 4,137,185. Particularly useful dienes are 5-ethylidene norbornene ; 1,4-hexadiene ; 2,5-norbornadiene and ethyl norbornadiene. The latter terpolymers are commercially available under the trademarks VISTALON® an elastomeric terpolymer of ethylene, propylene and 5-ethylidene norbornene, marketed by Exxon Chemical Company and NORDEL® a terpolymer of ethylene, propylene and 1,4-hexadiene, marketed by E. I. Du Pont de Nemours and Company

The term copolymer as used herein is meant to include terpolymers and tetrapolymers.

Other useful hydrocarbon substituents include styrene-isoprene copolymers, styrene-isobutene copolymers, isobutene-butadiene-1,3 copolymers, propene-isoprene copolymers, isobutene-chloroprene copolymers, isobutene-(para-methyl)styrene copolymers, copolymers of hexene-1 with hexadiene-1,3 copolymers of octene-1 with hexene-1, copolymers of heptene-1 with pentene-1, copolymers of 3-methyl-butene-1 with octene-1, copolymers of 3,3-dimethyl-pentene-1 with hexene-1, and terpolymers of isobutene, styrene and piperylene. More specificexamples of such interpolymers include copolymer of 95% (by weight) of isobutene with 5% (by weight) of styrene ; terpolymer of 98% of isobutene with 1% of piperylene and 1% of chloroprene ; terpolymer of 95% of isobutene with 2% of butene-1 and 3% of hexene-1 ; terpolymer of 60% of isobutene with 20% of pentene-1 and 20% of octene-1 ; copolymer of 80% of hexene-1 and 20% of heptene-1 ; terpolymer of 90% of isobutene with 2% of cyclohexene and 8% of propylene.

Techniques for attaching succinic anhydride groups to the above-described hydrocarbons and polymers are well known in the art and these include, for example, reacting maleic anhydride directly with the polyolefin which may have first been halogenated in the range of about 2 to 5 wt.% prior to the reaction of maleic anhydride. Grafting of maleic anhydride with ethylene propylene copolymers and terpolymers is described in U.S. Patents 4,089,794 and 4,137,185 and represents a particularly preferred embodiment since it provides a multi-site reactant which, upon aminolysis and cyclodehydration in accordance with the invention, results in a lubricating oil additive having especially valuable multi-functional dispersant-viscosity index improving properties and such products are especially useful in improving the dispersancy of diesel engine oil formuations.

Aza polycyclic ring assemblies (aza polycycles) useful in the invention contain 2 to 3 rings having 5 to 3 nitrogen atoms at least one being an NH group, but preferably 2-3 NH groups per molecule are present, which compounds can be represented by the formulas :

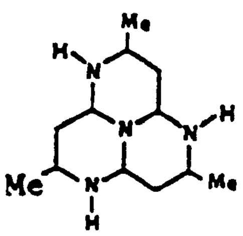 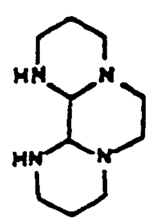 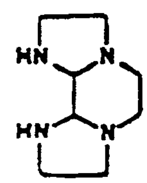 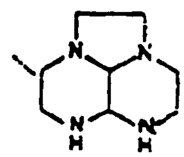

These amino compounds may be acylated by reacting at least a half-mole equivalent up to a 2 mole equivalent of the aforementioned hydrocarbon substituted succinic anhydrides to yield useful lubricant additives.

Further embodiments of the present invention reside in the formation of metal complexes and other post-treatment derivatives, e.g. borated derivatives, of the novel macrocyclic additives prepared in accordance with this invention. Suitable metal complexes may be formed in accordance with known techniques of employing a reactive metal ion species during or after the reaction of the polyamine and the hydrocarbyl anhydride compound. Complex-forming metal reactants include the nitrates, thiocyanates, halides, carboxylates, phosphates, thio-phosphates, sulfates, and borates of transition metals such as iron, cobalt, nickel, copper, chromium, manganese, molybdenum, tungsten, ruthenium, palladium, platinum, cadmium, lead, silver, mercury, antimony and the like. Prior art disclosures of these complexing reactions may be found in U.S. Patents 3,306,908 and Re. 26,433.

Post-treatment compositions include reacting the novel-additives of the present invention with one or more post-reacting reagents, usually selected from the group consisting of boron oxide, boron oxide hydrate, boron halides, boron acids, sulfur, sulfur chlorides, phosphorous sulfides and oxides, carboxylic acid or anhydride acylating agents, epoxides and episulfides and acrylonitriles. The reaction of such post-treating agents with the novel polyamine compounds of this invention is carried out using procedures known in the art. For example, boration is accomplished in accordance with the teaching of U.S. Patent 3,254,025 by treating the macrocyclic polyamine compound with a boron oxide, halide, ester or acid to provide about 0.1 to 1 atomic proportions of boron for each atomic proportion of nitrogen in the composition. Treatment is carried out by adding about 1-3 wt% of boron compound, preferably boric acid, and heating and stirring the reaction mixture at about 135°C to 165°C for 1 to 5 hours followed by nitrogen stripping and filtration, if desired. Mineral oil or inert organic solvents facilitate the process.

A particularly preferred post-treatment technique especially useful for preparation of lubricating oil dispersants is post-treatment with a polyisobutenyl succinic anhydride. In this embodiment a polyisobutenyl succinic anhydride-poly 3-aminopropylamine macrobicyclic reaction product is first prepared in accordance with the

invention, and subsequently treated with about 10-50 mole% of additional polyisobutenyl (Mn of about 900-2000) succinic anhydride at elevated temperatures of about 120°C for about one hour or until the reaction mixture shows complete reaction of the free anhydride. Such products are especially useful as dispersants for diesel engine lubricating oil compositions.

The products of this invention can be incorporated in lubricating oil compositions, e.g., gasoline or diesel crankcase lubricating oil in active ingredient concentrations within the range of about 0.01 to 20 wt% based on the total weight of the lubricating oil composition. Such additives are usually dispensed in the form of concentrates as discussed below.

The products prepared according to this invention can be incorporated in a wide variety of lubricants. They can be used in lubricating oil compositions, such as automotive crankcase lubricating oils, automatic transmission fluid, etc., in active ingredient concentrations generally within the range of about 0.5 to 10 wt%, for example, 1 to 5 wt.%, preferably 1.5 to 3 wt.% of the total composition when used only as a dispersant. Conventionally, the dispersants are admixed with the lubricating oils as concentrates which usually contain up to about 50% weight of the additive compound dissolved in mineral oil, preferably a mineral oil having an ASTM D-445 viscosity of about 2 to 40, preferably 5 to 20 centistokes at 99°C. The lubricating oil includes not only hydrocarbon oils derived from petroleum but also includes synthetic lubricating oils such as polyethylene oils ; alkyl esters of dicarboxylic acids, complex esters of dicarboxylic acid, polyglycol and alcohol ; alkyl esters of carbonic or phosphoric acids ; polysilicones ; fluorohydrocarbon oils ; and, mixtures of lubricating oils and synthetic oils in any proportion, et. The term "lubricating oil" for this disclosure includes all the foregoing. The useful additive of this invention may be conveniently dispensed as a concentrate of 10 to 80 wt.% such as about 49 wt.% of said dispersant in 20 to 90 wt.% of mineral oil, e.g., Solvent 150 Neutral oil with or without other additives being present.

As noted above, such composition or concentrates containing the dispersants of the present invention will also contain other well-known additives such as the zinc dialkyl ($C_3$-$C_8$) dithiophosphate anti-wear inhibitors usually present in amounts of from 1 to 5 wt.%.

Useful detergents include the normal basic or over-based metal, e.g., calcium magnesium, barium, etc., salts of petroleum naphthene acids, petroleum sulfonic acids, alkyl benzene sulfonic acids, oil-soluble fatty acids, alkyl salicyclic acids, alkylene bisphenols and hydrolyzed phosphosulfurized polyolefins. Typical amounts are from 1-7 wt.% with preferred materials being normal and basic calcium and magnesium sulfonates, phenates and sulfurized phenates.

Oxidation inhibitors include hindered phenols, e.g., 2,6-ditert-butyl para-cresol, amines, sulfurized phenols and alkyl phenothiazines, usually present in amounts of from 0.001 to 1 wt.%.

Pour point depressants usually present in amounts of 0.01 to 1 wt.% include wax alkylated aromatic hydrocarbons, olefin polymers and copolymers, acrylate and methacrylate polymers and copolymers.

Viscosity index improvers including olefin polymers and copolymers such as polybutene, ethylene-propylene copolymers, hydrogenated polymers and copolymers and terpolymers of styrene with isoprene and/or butadiene, polymers of alkyl acrylates or alkyl methacrylates, copolymers of alkyl methacrylates with N-vinyl pyrrolidone or dimethyl-aminoalkyl methacrylate, post-grafted polymers of ethylene-propylene with an active monomer such as maleic anhydride which may be further reacted with an alcohol or an alkylene polyamine, styrene/maleic anhydride polymers post-treated with alcohols and amines, etc. Such products are used in amounts as required to meet the viscosity grade desired, generally 1-15 wt.% being used.

Rust inhibition activity can be provided by the aforementioned metal dihydrocarbyl dithiophosphates and the corresponding precursor esters, phosphosulfurized pinenes, sulfurized olefins and hydrocarbons, sulfurized fatty esters and sulfurized alkyl phenols. Preferred are the zinc dihydrocarbyl dithiophosphates which are salts of dihydrocarbyl ($C_3$-$C_8$) esters of dithiophosphoric acids. Rust inhibitors are usually used in the range of 0.01 to 1 wt.%.

Fuel economy or friction reducing additives may also be present such as dimer acid esters with glycols as disclosed in U.S. Patent 4,105,781 issued to Shaub with the esters of dimerized linoleic acid and diethylene glycol being a preferred material or other effective fuel economy additives such as glycerol esters of $C_{16}$-$C_{18}$ fatty acids with glycerol oleate being especially suitable. These additives are present in amounts which vary greatly according to their effectiveness but generally are used in very small proportions.

Thus, the term lubricating oil composition as used herein is meant to include an oil of lubricating viscosity containing in addition to the products of this invention conventional additives in customary amounts as required to provide their normal attendant functions.

The polycyclic polyamine compounds of this invention may also be used in fuels in amounts sufficient to provide dispersant and detergent properties to the fuel, particularly in preventing and removing deposits from the carburetor and fuel lines when used preferably in gasoline fuel composition. The compounds of the invention may be used in fuels in amounts ranging from about 0.0001 to about 1 wt.%, preferably about 0.0004 to about

0.1 wt.% based on the weight of the fuel composition. Suitable fuel compositions include normally liquid hydrocarbon fuels such as motor gasoline (ASTM D439-73) diesel fuel or fuel oil (ASTM D-391) and mixtures of such hydrocarbon fuels with non-hydrocarbon materials such as alcohols, ethers, nitro compounds and the like.

The invention is further illustrated by the following examples which are not to be considered as limitative of its scope.

To emphasize the remarkable sensitivity of product composition to mode of addition, the prior art method, i.e. the addition of polyamine to hydrocarbon succinic anhydride (direct addition) is also described to illustrate the formation of linear imid product as taught in the prior art.

LINEAR DISPERSANT SYNTHESIS VIA DIRECT ADDITION ROUTE – COMPARATIVE EXAMPLES A1 AND 12

EXAMPLES A1. LINEAR POLYAMINE SUCCINIMIDE FORMATION VIA ADDITION OF 4,7-DIAZA-DECANE-1,10-DIAMINE TO POLYISOBUTENYL-SUCCINIC ANHYDRIDE (1 : 1 MOLAR RATIO)

About 200 g (ca. 0.087) mole of polyisobutenyl succinic anhydride (PIBSA) prepared via Ene reaction of polyisobutylene ($\bar{M}n$ 1300 by vapor phase osmometry) and maleic anhydride as described by Brois and Gutierrez in U.S. Patent 4,239,636, and having a saponigication number of 48.5 were charged into a reaction flask and heated to 150°C and stirred under a nitrogen atmosphere. About an equimolar quantity (15.1 g) of 4,7-diaza-decane-1,10-diamine were added dropwise to the neat PIBSA reactant at 150°C over a 15 minute period. After polyamine treatment, the reaction mixture was sparged with nitrogen gas at 150°C for about two hours, and subsequently filtered. The filtrate featured an infrared spectrum with a strong imide carbonyl absorption band at about 5.9 microns ascribable to linear polyamine imides of PIBSA. The analyses indicate that the product is a mixture of 1(2-polyisobutenylsuccinimido)-4,7-diazadecane-10-amine and 1,10-bis-(2-polyisobutenylsuccinimodo)-4,7-diazadecane. The filtered residue analyzed for 1.93% N, and 0.24 milliequivalent of primary amine per gram of product.

EXAMPLES A2 LINEAR POLYAMINE BIS-IMIDE VIA ADDITION OF 4,7-DIAZA-DECANE-1,10-DIAMINE TO POLYISOBUTENYLSUCCINIC ANHYDRIDE (1 : 2 MOLAR RATIO)

About 200 g (ca. 0.087 mole) of the PIBSA described in Example A1 were charged into a reaction flask and heated to about 150°C and stirred under a nitrogen atmosphere. Thereafter, 7.6 g (ca. 0.043 mole) of 4,7-diaza-decane-1,10-diamine were added dropwise over a 15 minute period while the reaction temperature was maintained at about 150°C. Once the polyamine addition was complete, the reaction mixture was sparged with gaseous nitrogen for about two hours at 150°C, and subsequently filtered. The filtered product featured an infrared spectrum with a dominant imide carbonyl absorption band at about 5.9 microns, analyzed for 1.17% N, and 00.00 meq of primary amine per gram of product. The analytical data are consistent with bis-imide product which can be described mainly as 1,10-bis-(2-polyisobutenylsuccinimido)-4,7-diazadecane.

B. EXAMPLES OF DISPERSANTS PREPARED VIA AMINATION OF PIBSA WITH AZA POLYCYCLIC COMPOUNDS.

EXAMPLE B1. AMINOLYSIS OF PIBSA WITH 3,7,11-TRIMETHYL-2,6,10,13-TETRAAZA-TRICYCLO (7.3.1.05.13) TRIDECANE(TTT).

Approximately 115 gms (0.05 moles) of PIBSA of Example A1 and 11.2 gms (0.05 moles) of TTT were dissolved in 100 mls xylene. The mixture was refluxed 2 hours, nitrogen sparged and filtered to give an acylated aza polycyclic product which analyzed for 1.05% nitrogen.

EXAMPLE B2. AMINOLYSIS OF PIBSA WITH 1,4,8,11-TETRAAZATRICYCLO-(8.4.0.04,9) TETRADECANE (TATT).

173 gms (0.1 mole) of PIBSA obtained by the thermal route with molecular weight (by VPO) of 1300 and saponification number of 65 and 19.6 gms (0.1 mole) of TATT were combined and heated at 180°C for 2 hours, nitrogen sparged for 1 hour at 180°C. The acylated aza polycyclic product analyzed for 2.76% nitrogen.

C. DISPERSANT-VI IMPROVERS VIA AMINATION OF EPSA WITH AZA POLYCYCLIC COMPOUNDS

EXAMPLE C1. MODIFICATION OF EPSA WITH 2,12-DIMETHYL-2,6,10-TRIAZABICYCLO(8.3.0) TRIDEC-1-EN-11-ONE(TBT).

An 11% mineral oil (Solvent 100 Neutral) solution of EP copolymer of Mn-50,000 was grafted with maleic anhydride by heating in the presence of peroxide as shown in Example 1 of U.S. Patent 4,089,794 to provide an EPSA having an acid value of about 0.14 meg, per gram of plymer.

3.4 gms (15.2 millimoles) of TBT was dissolved in 100ml of xylene and mixed with 55 gms of this EPSA ; the reaction mixture was gradually heated to 150° for 1 hour to distill off xylene and water of reaction, then sparged with nitrogen at 160° for 1 hour. The acylated aza crown compound analyzed for 0.87% nitrogen.

EXAMPLE C2. AMINATION OF EPSA WITH 3,7,11-TRIMETHYL-2,6,10,13-TETRAZAZATRICYCLO $[7.3.1.0^{5,13}]$ TRIDECANE(TTT).

100 gms of EPSA of Example C1 and 3.1 gms (0.014 moles) of TTT were combined and heated at 180° for 2 hours, nitrogen sparged at 180°C for 1 hour and cooled. The acylated azapolycyclic product analyzed for 0.43% nitrogen.

C3. AMINATION OF EPSA WITH 1,4,8,11-TETRAAZATRI CYCLO$[8.4.0.0^{4,9}]$TETRADECANE(TATT)

100 gms of EPSA of Example C1 were mixed with 2.7 gms (13.8 millimoles) of TATT, heated to about 200° and maintained at this temperature for 2 hours with nitrogen sparging. The acylated aza polycyclic product contained 1.05% nitrogen.

EXAMPLE C4. AMINATION OF EPSA WITH 1,4,7,9-TETRAAZATRICYCLO$[6.3.1.0^{4,12}]$-DODECANE (TATD).

100 gms of EPSA of Example C1 were mixed with 2.4 gms (14.3 millimoles) of TATD and gradually heated to 200°C. The mixture was maintained at 200°C with nitrogen sparging for 2 hours. The acylated aza polycyclic product analyzed for 1.0% nitrogen.

In Tables 1 and 2 below the products of this invention were evaluated for sludge varnish potency in the SIB (Sludge Inhibition Bench Test) and VIB (Varnish Inhibition Bench Test). Comparative Tests with prior art products were also conducted. Tests are described below :

The SIB test employs a used crankcase mineral lubricating oil composition having an original viscosity of about 325 SUS at 37.8°C that has been used in a taxicab that was driven generally for short trips only, thereby causing a buildup of a high concentration of sludge precursors. The oil that was used contained only a refined base mineral oil, a viscosity index improver, a pour point depressant and zinc dialkyldithiophosphate anti-wear additive. The oil contained no sludge dispersants. The quantity of such used oil was acquired by draining and refilling the taxicab crankcase at 1,000-2,000 mile intervals.

The SIB test is conducted in the following manner : The used crankcase oil is freed of sludge by centrifuging for one half hour at about 39,000 gravities (gs). The resulting clear bright red oil is then decanted from the insoluble sludge particles thereby separated out. However, the supernatant oil still contains oil-soluble sludge precursors which under the conditions employed by this test will tend to form additional oil-insoluble deposits of sludge. The sludge inhibiting properties of the additives being tested are determined by adding to portions of the used oil, a small amount of the particular additive being tested. Ten grams of each one being tested is placed in a stainless steel centrifuge tube and is heated at 137.8°C for 16 hours at the presence of air. Following the heating, the tube containing the oil being tested is cooled and then centrifuged for 30 minutes at about 39,000 gs. Any deposits of new sludge that forms in this step are separated from the oil by decanting supernatant oil and then carefully washing the sludge deposits with 15 ml. of pentane to remove all remaining oils from the sludge. The weight of the new solid sludge that formed in test, in milligrams, is determined by drying the residue and weighing it. The results are reported as milligrams of sludge per ten grams of oil, thus measuring differences as small as one part per ten thousand. The less new sludge formed, the more effective is the additive as a dispersant. In other words, if the additive is effective, it will hold at least a portion of the new sludge that forms on heating and oxidation stably suspended in the oil so that it does not precipitate down during the centrifuging period.

In the VIB test, a test sample consisting of ten grams of lubricating oil containing the additive being evaluated is used. The test oil is a commercial lubricating oil obtained from a taxi after two thousand miles of

driving with said lubricating oil. Each sample is heat soaked overnight at about 140°C and thereafter centrifuged to remove the sludge. The supernatant fluid of each sample is subjected to heat cycling from about 150°C to room temperature over a period of 3.5 hours at a frequency of about two cycles per minute. During the heating phase, a gas containing a mixture of 0.7 volume percent $SO_2$, 1.4 volume percent NO and the balance air was bubbled through the test samples and during the cooling phase, water vapor was bubbled through the test samples. At the end of the test period, which testing cycle can be repeated as necessary to determine the inhibiting effect of any additive, the wall surfaces of the test flasks in which the samples were contained are visually evaluated as to the varnish inhibition. The amount of varnish imposed on the walls is rated at values of from one to seven with the higher number being the greater amount of varnish. It has been found that this test correlates with the varnish results obtained as a consequence of carrying out ASTM-MS-VC engine tests.

EXAMPLE D1. EVALUATION OF DISPERSANTS IN THE SIB AND VIB TESTS

A series of SIB and VIB evaluations were carried out comparing macrobicyclic and polycyclic polyamine dispersants of this invention with non-cyclic linear bis-imide dispersants prepared utilizing the prior art conditions which do not provide the macrocyclic structure.

Included in the testing for comparative purposes are two dispersants CD-1 and CD-2 which are representative of current commercially available products and are made by aminating PIBSA of Mn 900 and 1300 respectively with tetraethylene pentamine to give polyisobutenyl succinimide products which are linear bis-imide structures. These results are in the table below.

TABLE 1 - EXAMPLE D1

SIB RESULTS

| ADDITIVE EXAMPLE | TYPE | MG SLUDGE/10.0g Oil | VIB RATING |
|---|---|---|---|
| EX A-2 | L | 2.93 | 5 |
| EX B-2 | PC | 0.48 | 2 |
| CD-1 | L | 4.54 | 5 |
| CD-2 | L | 5.29 | 5 |

EXAMPLE D2. EVALUATION OF DISPERSANT VI IMPROVERS IN THE SIB AND VIB TESTS.

A number of products of the invention as prepared in the foregoing examples are suitable as dispersant – VI improvers were evaluated in the SIB/VIB tests and compared for activity against a compound representative of current commercially suitable products. The results are tabulated below.

In the table, PC represents the structural categorization of the products of the invention, i.e. polycyclic polyamine dispersant – VI improvers. CD-3 is a product which qualifies under commercial standards and is made by reaction of EPSA (Ex. C1) and N-(3-aminopropyl) morpholine, and is included for comparative purposes.

TABLE 2 - EXAMPLE D-2

SIB RESULTS

| ADDITIVE EXAMPLE | TYPE | MG SLUDGE/10.0g Oil | VIB RATING |
|---|---|---|---|
| C2 | PC | 1.25 | 2 - 2.5 |
| C3 | PC | 2.37 | 2 |
| C4 | PC | 2.09 | 2 |
| CD-3 | IMIDE | 6.29 | 5 |

## Claims

1. An oil soluble polycyclic polyamine compound being the reaction product of a hydrocarbon succinic anhydride or carboxylic acid having 8 to 15,000 carbon atoms with an aza polycyclic ring compound containing 2 to 3 rings having 5 or 6 atoms per ring including 3 to 4 nitrogen atoms at least one of which is an NH group.

2. A lubricating oil composition comprising a major amount of an oil of lubricating viscosity and 0.01 to 20 wt% of the macrocyclic compounds of claim 1.

3. A fuel composition comprising a major amount of a hydrocarbon fuel containing about 0.001 to about 1 wt% of the compound of claim 1.

4. The use as an additive for lubricating oil of a compound according to claim 1.

5. The use as an additive for fuels of a compound according to claim 1.

## Ansprüche

1. Öllösliche polycyclische Polyaminverbindung, die das Reaktionsprodukt von Kohlenwasserstoffbernsteinsäureanhydrid oder -carbonsäure mit 8 bis 15000 Kohlenstoffatomen mit einer polycyclischen Azaringverbindung mit 2 bis 3 Ringen mit 5 oder 6 Atomen je Ring einschließlich 3 bis 4 Stickstoffatomen, von denen mindestens eines eine NH-Gruppe ist, ist.

2. Schmierölzusammensetzung, die eine überwiegende Menge eines Öls mit Schmierviskosität und 0,01 bis 20 Gew.% der makrocyclischen Verbindungen gemäß Anspruch 1 enthält.

3. Brennstoffzusammensetzung, die eine überwiegende Menge eines Kohlenwasserstoffbrennstoffs umfaßt, der etwa 0,001 bis etwa 1 Gew.% der Verbindung von Anspruch 1 enthält.

4. Verwendung einer Verbindung gemäß Anspruch 1 als Additiv für Schmieröl.

5. Verwendung einer Verbindung gemäß Anspruch 1 als Additiv für Brennstoffe.

## Revendications

1. Composé polyaminé polycyclique soluble dans l'huile, qui est le produit de réaction d'un anhydride succinique à substitution hydrocarbonée ou d'un acide carboxylique ayant 8 à 15000 atomes de carbone avec un composé à noyau polycyclique aza contenant 2 ou 3 cycles ayant 5 ou 6 atomes par cycle, renfermant 3 ou 4 atomes d'azote dont au moins l'un fait partie d'un groupe NH.

2. Composition d'huile lubrifiante comprenant une forte quantité d'une huile de viscosité propre à la lubrification et 0,01 à 20% en poids du composé macrocyclique suivant la revendication 1.

3. Composition de carburant comprenant une forte quantité d'un carburant hydrocarboné contenant environ 0,001 à environ 1% en poids du composé suivant la revendication 1.

4. Utilisation comme additif pour une huile lubrifiante d'un composé suivant la revendication 1.

5. Utilisation comme additif pour carburants d'un composé suivant la revendication 1.